# EUROPEAN PATENT APPLICATION

(11) **EP 2 884 248 A2**
(43) Date of publication of application: **17.06.2015**
(21) Application number: 14160461.1
(22) Date of filing: 18.03.2014
(51) Int. Cl.: G01J 5/00, G01J 5/08, G01J 5/02

(54) **Forehead thermometer**

(30) Priority: 12.12.2013 CN 201310683151
(71) Applicant: Microlife Corporation, Taipei (TW)
(72) Inventor: Ho, Chia-Chen, Taipei (TW)
(74) Representative: Viering, Jentschura & Partner Patent- und Rechtsanwälte

(57) **Abstract**

A forehead thermometer has a body (10), a detecting unit (20) and a point light source (40). The detecting unit (20) and the point light source (40) are mounted adjacent to each other and are mounted in a front end (101) of the body (10). A first convex lens (30) is mounted between the detecting unit (20) and the front end (101) of the body (10) to focus the infrared energy from the forehead to the detecting unit (20) so that the measuring precision is enhanced and also provides focusing effect within a limited space. Therefore, the forehead thermometer is lighter and smaller.

## Description

### 1. Field of the Invention

The present invention relates to a forehead thermometer, especially to a non-contact forehead thermometer that measures a forehead temperature or a core temperature of a human body.

### 2. Description of the Prior Arts

Forehead thermometers are widely used to measure body temperature of a human body at the forehead. The conventional forehead thermometer has an infrared detecting unit mounted in a front end of a body of the conventional forehead thermometer. The infrared detecting unit receives the infrared energy radiated from the forehead and transmits the detecting result to a computing unit of the conventional forehead thermometer to compute and convert the infrared energy into temperature to estimate a forehead temperature or a core temperature of a human body.

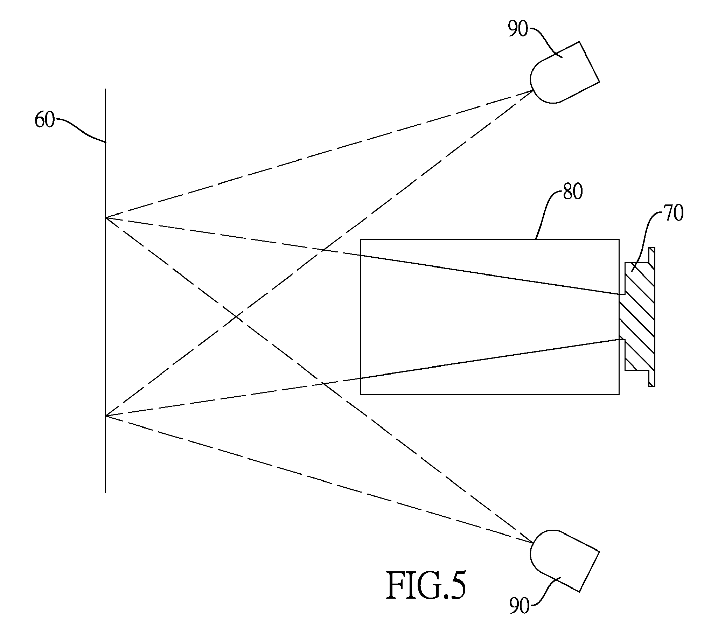

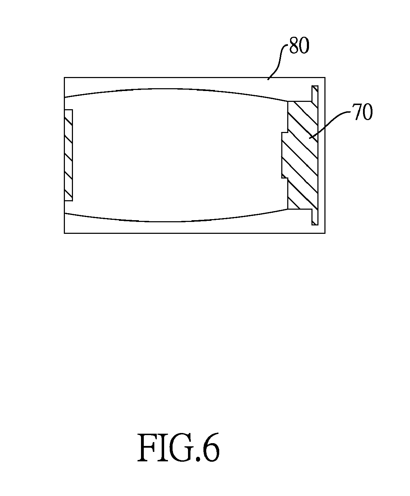

Because the area of the forehead is relatively large and the forehead is surrounded by many non-skin parts such as eyebrows, hair, eyes and so on, the forehead temperature or a core temperature of a human body is not properly measured if the detecting range covers any of the non-skin parts. Therefore, a conventional forehead thermometer shown in Figs. 5 and 6 further comprises a focusing tube 80 and two point light sources 90. The focusing tube 80 is mounted in front of the detecting unit 70 and has a straight sidewall or an arc sidewall to reflect the infrared ray. The point light sources 90 are mounted respectively on opposite sides of the detecting unit 70. When the conventional forehead thermometer is used, the lights from the point light sources 90 are emitted onto the forehead of the user. The user holds the conventional forehead thermometer to move slowly until the lights from the point light sources 90 are emitted at the same point on the forehead 60. When the same point is emitted on the forehead 60, the detecting unit 70 is located on a best detecting position and has a best detecting distance from the forehead 60. The infrared energy from the forehead is reflected by the focusing tube 80 and is received by the detecting unit 70. After detecting for a few seconds, the measuring procedure is completed. Thus, the point light sources 90 are used to get the best measuring position and distance, and the focusing tube 80 is used to focus the infrared energy.

The conventional forehead thermometer needs the focusing tube 80 to focus the infrared energy, but the focusing tube 80 has limited focusing effect and occupies a certain length at the front part of the conventional forehead thermometer. Therefore, the conventional forehead thermometer cannot precisely measure the core temperature of the human body and the focusing tube obstructs the conventional forehead thermometer from being lighter and smaller.

To overcome the shortcomings, the present invention provides a forehead thermometer to mitigate or obviate the aforementioned problems.

The main objective of the present invention is to provide a forehead thermometer that has an improved measuring precision and occupies less space.

To achieve the main objective, a forehead thermometer in accordance with the present invention comprises a body having a detecting opening formed on a front end of the body and a light opening formed on the front end of the body; a detecting unit mounted in the front end of the body and aligning with the detecting opening; a first convex lens mounted in the front end of the body and adhered to the detecting unit; a point light source mounted in the front end of the body and aligning with the light opening; and a second convex lens mounted in the front end of the body and mounted between the light opening and the point light source. The point light source emits different lights in at least two colors.

The forehead thermometer in accordance with the present invention has the following advantages. With the first convex lens focusing the energy to transmit to the detecting unit to replace the focusing tube that occupies larger space. The detecting unit is directly adhered to the first convex lens, the forehead thermometer of the present invention has an enhanced focusing effect and also occupies smaller space to decrease the volume and the length of the body. The measuring precision of the detecting unit is also improved, and the forehead thermometer in accordance with the present invention is also lighter and smaller. Further, since the optical filter is mounted in the detecting unit, the visible light is filtered to keep the detecting unit from interference by the visible light.

Other objectives, advantages and novel features of the invention will become more apparent from the following detailed description when taken in conjunction with the accompanying drawings.

### IN THE DRAWINGS

Fig. 1 is a perspective view of a forehead thermometer in accordance with the present invention;
Fig. 2 is a side view in partial section of the forehead thermometer in Fig. 1;
Fig. 3 is an enlarged operational side view in partial section of the forehead thermometer in Fig. 1;
Fig. 4 is an operational perspective view of the forehead thermometer in Fig. 1;
Fig. 5 is an operational enlarged illustrative side view in partial section of a conventional forehead thermometer in accordance with the prior art; and
Fig. 6 is an enlarged illustrative side view in partial section of another conventional forehead thermometer in accordance with the prior art.

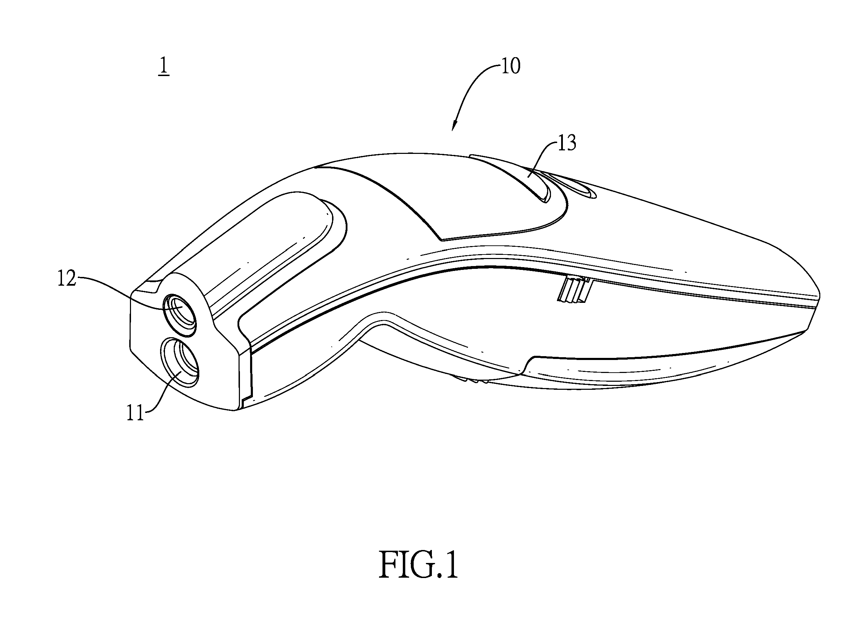

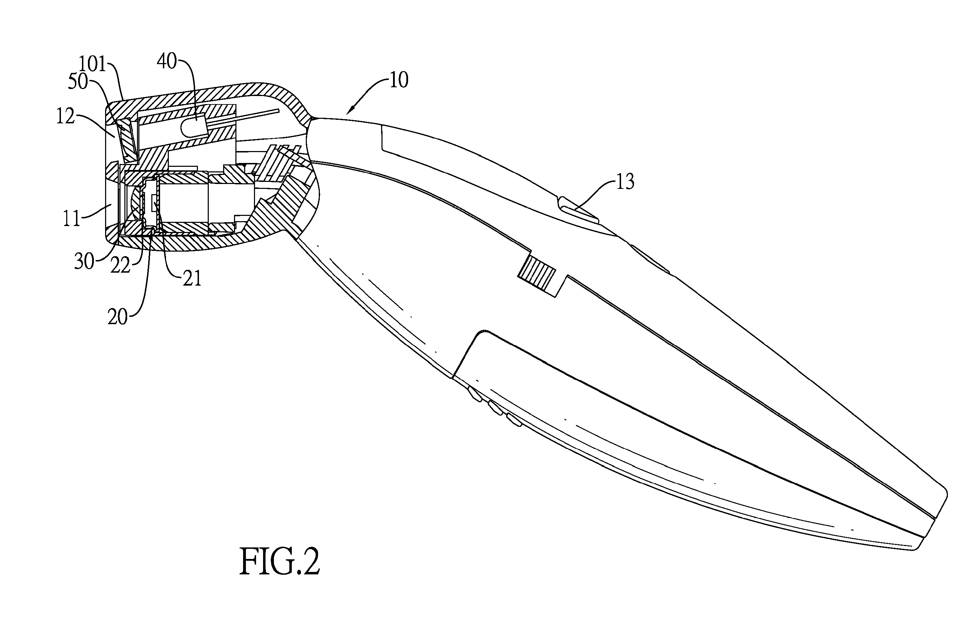

With reference to Figs. 1 and 2, a forehead thermometer in accordance with the present invention comprises a body 10 with a switch 13, a detecting unit 20, a first convex lens 30, a point light source 40 and a second convex lens 50.

The body 10 has a detecting opening 11 and a light opening 12 formed on a front end 101 of the body 10 and adjacent to each other. In a preferred embodiment, the light opening 12 is formed on, but not limited to, a top side of the detecting opening 11.

The detecting unit 20 is mounted in the front end 101 of the body 10, aligns with the detecting opening 11, and has a sensor 21 and an optical filter 22. The sensor 21 is mounted on a rear end of the detecting unit 20. The optical filter 22 is adhered to an inner side of a front end of the detecting unit 20 and is mounted between the sensor 21 and the detecting opening 11.

The first convex lens 30 is mounted in the front end 101 of the body 10 and is mounted between the detecting opening 11 and the detecting unit 20.Specifically, the first convex lens 30 is adhered to an outer side of the front end of the detecting unit 20. In a preferred embodiment, the first convex lens 30 may be, but is not limited to, a double convex lens, a Plano-convex lens, or a concave-convex lens.

The point light source 40 is mounted in the front end 101 of the body 10 and aligns with the light opening 12. The point light source 40 may emit different lights in at least two colors. The point light source 40 is a light emitting diode (LED).

The second convex lens 50 is mounted in the front end 101 of the body 10 and is mounted between the light opening 12 and the point light source 40 to further focus the light from the point light source 40.

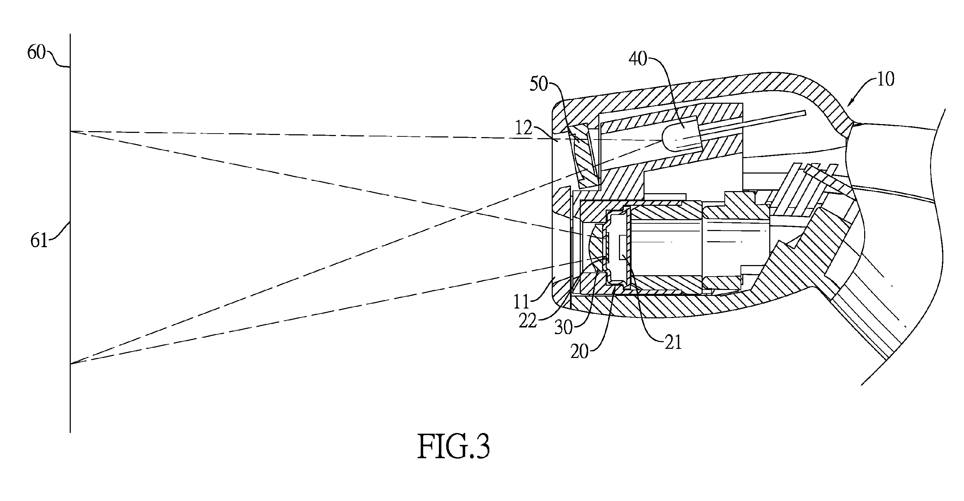

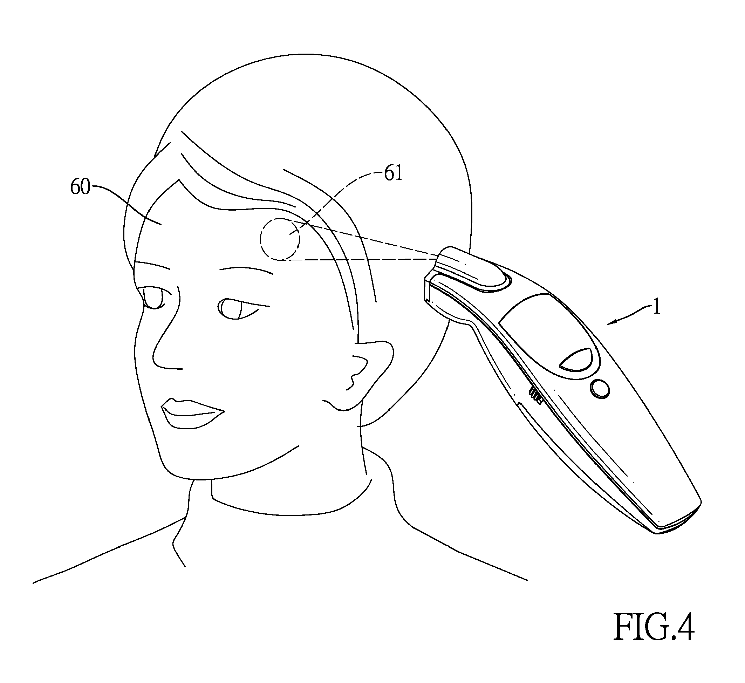

With reference to Figs. 3 and 4, when the forehead thermometer as described is used, the front end 101 of the body 10 is placed approaching the forehead 60 of the user and the switch 13 is pressed to actuate the detecting unit 20 and the point light source 40. The emitting range 61 of the point light source 40 is changed with the adjusted distance between the point light source 40 and the forehead 60 of the user, which means that the emitting range 61 of the point light source 40 is enlarged or narrowed while the body 10 is moved relative to the forehead 60. Because the emitting range 61 refers to the measuring range of the detecting unit 20, the body 10 is moved to find a preferred emitting range 61. When the preferred emitting range 61 is found, the body 10 is stopped moving. The infrared energy from the emitting range 61 on the forehead 60 is focused by the first convex lens 30 and is received by the detecting unit 20. After the body 10 is held at the preferred measuring position for 1 to 4 seconds, preferably for 3 seconds, the body 10 notifies the user that the measurement is completed by light signals or alarms. The detecting unit 20 transmits the measuring data to a computing unit in the body 10 to convert the measuring data into the temperature. Then the body 10 outputs the measured temperature of the user by display or voice. In a preferred embodiment, if the measured temperature of the user is over 37.5 degrees centigrade, light emitted from the point light source 40 changes to another color to notify the user that the user may have fever.

The forehead thermometer as described has the following advantages. Because the first convex lens 30 focuses the energy to transmit to the detecting unit 20 to replace the focusing tube that occupies larger space and is directly adhered to the detecting unit 20, the forehead thermometer of the present invention has an enhanced focusing effect and also occupies smaller space to decrease the volume and the length of the body 10. The measuring precision of the detecting unit 20 is also improved. Further, since the optical filter 22 is mounted in the detecting unit 20, the visible light is filtered to keep the detecting unit 20 from interference by the visible light.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A forehead thermometer **characterized in that**:
a body (10) has
a detecting opening (11) formed on a front end (101) of the body (10); and
a light opening (12) formed on the front end (101) of the body (10);
a detecting unit (20) is mounted in the front end (101) of the body (10) and aligning with the detecting opening (11);
a first convex lens (30) is mounted in the front end (101) of the body (10) and adhered to the detecting unit (20);
a point light source (40) is mounted in the front end (101) of the body (10) and aligning with the light opening (12); and
a second convex lens (50) is mounted in the front end (101) of the body (10) and mounted between the light opening (12) and the point light source (40).

2. The forehead thermometer as claimed in claim 1, wherein the first convex lens (30) is a double convex lens.

3. The forehead thermometer as claimed in claim 1, wherein the detecting unit (20) has a sensor (21).

4. The forehead thermometer as claimed in claim 3, wherein the detecting unit (20) has an optical filter (22) mounted between the detecting opening (11) and the sensor (21).

5. The forehead thermometer as claimed in claim 1, wherein the point light source (40) emits different lights in at least two colors.

6. The forehead thermometer as claimed in claim 1, wherein the light opening (12) is formed on a top side of the detecting opening (11).

7. The forehead thermometer as claimed in claim 1, wherein the point light source (40) is a light emitting diode.

8. The forehead thermometer as claimed in claim 1, wherein the first convex lens (30) is a Plano-convex lens.

9. The forehead thermometer as claimed in claim 1, wherein the first convex lens (30) is a concave-convex lens.
